# FASCICULE DE BREVET EUROPEEN

(11) **EP 2 892 483 B1**
(45) Date de publication et mention de la délivrance du brevet: **08.11.2017**
(21) Numéro de dépôt: 13766612.9
(22) Date de dépôt: 03.09.2013
(51) Int. Cl.: A61F 13/06, A61F 13/02

(54) **NOUVELLE BANDE ELASTIQUE AUX PROPRIETES DE CONTENTION OPTIMISEES**
NEUARTIGE ELASTISCHE BANDAGE MIT VERBESSERTEN STÜTZEIGENSCHAFTEN
NOVEL ELASTIC BANDAGE HAVING IMPROVED SUPPORT PROPERTIES

(30) Priorité: 03.09.2012 FR 1258185
(43) Date de publication de la demande: 15.07.2015
(73) Titulaire: Urgo Recherche Innovation et Développement, 21300 Chenove (FR)
(72) Inventeur: PERNOT, Jean-Marc, Henri, Maurice, F-21000 Dijon (FR); LECOMTE, Serge, F-21000 Dijon (FR)
(74) Mandataire: Chantraine, Sylvie Hélène
(86) Numéro de dépôt international: PCT/FR2013/052025
(87) Numéro de publication internationale: WO 2014/033418

(56) Documents cités:
- EP-A1- 2 058 424
- FR-A1- 2 890 854
- US-A- 5 939 339
- US-A1- 2003 040 691
- US-A1- 2011 208 101

## Description

La présente invention est relative à une nouvelle bande de contention, constituée par l'assemblage d'un matériau élastique et d'un non tissé autoadhérent à base de fibres courtes conjuguées qui ont été frisées, dont l'efficacité augmente au cours du temps.

L'invention concerne également l'utilisation de cette bande, seule ou dans un système de contention bicouches, notamment pour la prévention ou le traitement des pathologies veineuses ou des lymphoedèmes.

L'utilisation de divers systèmes de contention est connue depuis longtemps pour traiter les pathologies d'origine veineuse, comme par exemple l'insuffisance veineuse, le traitement des varices et des ulcères de jambes ou encore pour prévenir la thrombose veineuse ou le traitement des lymphoedèmes.

Ces systèmes sont constitués d'une ou de plusieurs bandes élastiques qui appliquent une pression sur le membre à traiter.

L'application d'une pression adéquate agit favorablement:
- d'une part, au niveau des vaisseaux en réduisant le calibre des veines ce qui conduit à l'accélération du flux sanguin et au rétablissement de la fonction valvulaire; et
- d'autre part, au niveau des tissus en favorisant une meilleure oxygénation et une résorption de l'oedème.

Dans le traitement des plaies chroniques et tout particulièrement dans celui des ulcères de jambes, l'utilisation d'un système de contention - qui permet de rétablir ou de favoriser une circulation veineuse normale - est le traitement de référence. C'est la seule thérapie qui ait prouvé son efficacité pour soigner et éviter la récidive de ce type de plaies.

Un système de contention efficace doit permettre de répondre à quatre objectifs principaux.

Premièrement, le système doit pouvoir être porté de façon continue jour et nuit durant un ou plusieurs jours (par exemple une semaine) selon la pathologie, sa gravité ou l'objectif thérapeutique (traitement ou prévention).

A cet effet, ce système doit donc permettre d'appliquer simultanément :
- d'une part, une pression relativement faible appelée «pression de repos», quand le muscle est relâché pour être confortable et en particulier supportable durant la nuit; et
- d'autre part, une pression relativement élevée appelée «pression de travail», quand le muscle est tendu ou lors des mouvements, et en particulier pendant la marche.

Deuxièmement, le différentiel de pression entre pression de repos et pression de travail doit être suffisant pour favoriser le reflux veineux.

Troisièmement, les valeurs de pression au repos, de pression de travail et de différentiel de pression doivent être stables dans le temps.

Quatrièmement, le système doit être facile et rapide à poser, et ceci en toute sécurité, afin d'éviter les risques de garrot si la pression appliquée est trop élevée, ou d'inefficacité si la pression ou le différentiel de pression est trop faible.

Pour parvenir à ces objectifs, on a développé des bandes élastiques tricotées ou tissées appelées bandes de contention.

Lors de son application autour d'un membre comme la jambe, la bande est étirée; suivant le degré d'allongement, elle applique sur la jambe une pression plus ou moins élevée. Cette pression, qui est la pression de traitement, dépend principalement de deux facteurs, l'allongement de la bande à la pose et la circonférence du membre sur lequel cette dernière est appliquée.

Les bandes élastiques de contention sont donc enroulées à un allongement donné autour de la jambe. Lors de l'enroulement de la bande, on procède à un recouvrement plus ou moins total de celle-ci sur elle-même. Très souvent, ce recouvrement est de 50 % dans le sens de pose transversal de la bande.

On considère généralement qu'un différentiel de pression à 24 heures compris entre 15 et 25 mm de mercure est nécessaire pour rétablir un flux veineux correct. Toutefois selon la pathologie, qu'il s'agisse d'un traitement sur des jambes sans ulcère grave, d'un traitement difficile sur des jambes abimées par un oedème, ou d'un traitement d'un ulcère mixte artériel et veineux, cette plage de valeurs peut s'étendre de 10 à 35 mm de mercure voire même de 10 à 40 mm de mercure.

Pour les ulcères, on vise généralement une pression de travail appliquée à 24 heures comprise entre 20 et 60 mm de mercure selon les pathologies.

Dans le cas du traitement des lymphoedèmes, l'élément important est davantage la pression appliquée que le différentiel de pression, et l'on vise pour cette pathologie, par exemple pour le lymphoedème de la jambe, une pression de travail à 24 heures supérieure à 60 mm de mercure, de préférence comprise entre 65 et 100 mm de mercure.

Les fabricants fournissent généralement des tableaux et des gammes de produits qui, pour un diamètre de membre donné, permettent de déterminer la pression à appliquer et de choisir le système adéquat.

Les bandes de contention utilisées sont classées par les spécialistes de la contention en deux grandes catégories selon la mesure de leur allongement

La classification est basée sur la mesure de l'allongement telle que définie dans la norme EN 14704-1 quand la bande est soumise à une force de traction maximale de 6 N/cm.

Les conditions de réalisation de la mesure sont les suivantes.

Une éprouvette du matériau à tester de 50 mm de largueur et de 250 à 300 mm de longueur est découpée et positionnée sans précontrainte dans les mors d'un dynamomètre électronique (par exemple un dynamomètre de marque MTS) de sorte à avoir une largeur de 50 mm et une longueur utile de référence de 200 mm. Le dynamomètre étire l'éprouvette à une vitesse de 100 mm/min jusqu'à une force maximale de 6 N/cm puis la traverse revient à sa position initiale à la même vitesse de retour de 100 mm/min. Ce cycle est effectué 5 fois et l'allongement obtenu au cinquième cycle, exprimé en pourcentage, est directement calculé par l'appareillage. L'opération est répétée sur 5 éprouvettes et on prend la valeur moyenne.

### 1) Les bandes dites à allongement court

Sur la base de ce test pris comme référence, on considère qu'une bande de contention est une bande à «allongement court» si son allongement est inférieur ou égal à 100%.

Ces bandes exercent une pression au repos basse et une pression de travail élevée. Elles ont donc un grand différentiel de pression en particulier lors des mouvements, par exemple pendant la marche.

Les bandes à allongement court possèdent toutefois de nombreux inconvénients.

Tout d'abord, elles sont difficiles à poser car de faibles variations d'allongement à la pose génèrent de fortes augmentations ou diminutions de la pression atteinte et du différentiel de pression obtenu. Il existe donc un risque de garrot si la pression appliquée est trop élevée, ou un risque d'inefficacité si elle est insuffisante.

On constate aussi une diminution significative au cours du temps de la pression appliquée et du différentiel de pression entre pression de travail et pression de repos, et souvent un glissement de la bande de contention.

Il en résulte la nécessité de procéder à des changements fréquents de ces bandes et une augmentation corrélative du coût du traitement.

Une bande à allongement court est par exemple commercialisée par la société ACTIVA sous la dénomination Actico®. Cette bande est enroulée sur une bande de ouate préalablement enroulée sur la jambe. La ouate est destinée à répartir les pressions à la surface du membre, et/ou à protéger par son épaisseur les saillies osseuses, et à absorber les éventuels exsudats si la bande est posée sur une plaie ouverte, par exemple dans le cas des ulcères de jambes.

### 2) Les bandes à allongement long

Sur la base du test précédent pris comme référence, on considère qu'une bande est une bande à «allongement long» si son allongement est supérieur à 100%.

Ces bandes sont plus faciles à poser car elles présentent une plus grande extensibilité. De ce fait, des variations d'allongement à la pose ne génèrent pas de variations importantes de la pression appliquée. Le risque de garrot est faible.

En revanche, ces bandes conduisent à de faibles variations de pression entre repos et travail, et à une faible variation de pression au cours des mouvements, par exemple lors de la marche. Elles s'avèrent moins efficaces que les bandes à allongement court.

Elles présentent aussi un certain inconfort en position de repos si l'on souhaite imposer une pression élevée, d'où la nécessité de les retirer la nuit en raison de la gêne occasionnée.

Des bandes à allongement long sont par exemple commercialisées par les sociétés THUASNE et SMITH ET NEPHEW respectivement sous les dénominations Biflex® et Proguide®.

Pour essayer de remédier à ces inconvénients, des systèmes multicouches ont été développés.

Tous les systèmes de contention aujourd'hui utilisés sont constitués soit d'une bande unique choisie parmi ces deux catégories, soit de l'association de plusieurs bandes choisies parmi ces deux catégories, si nécessaire en association avec une première couche de ouate en contact avec la peau.

Les systèmes de contention commercialisés les plus performants aujourd'hui sont des systèmes bicouches. De tels systèmes sont par exemple commercialisés par la société Laboratoires URGO sous les dénominations K2® et K2® Lite. La première bande (commercialisée sous la dénomination Ktech®) est une bande à allongement court qui applique l'essentiel de la pression, constituée d'une couche de ouate aiguilletée à un tricot élastique. La deuxième bande (commercialisée sous la dénomination Kpress®) est une bande à allongement long autoadhérente, qui applique la pression complémentaire par rapport à la première bande pour obtenir la pression recherchée, et qui sert à maintenir la première bande en place. La deuxième bande est constituée d'un tissé enduit de latex. Ce système permet d'optimiser la conservation de la pression au cours du temps et du différentiel de pressions appliquées.

En travaillant sur une meilleure optimisation de l'efficacité de ces systèmes de contention, notamment pour améliorer la conservation au cours du temps i) des pressions appliquées, même quand elles sont élevées comme dans le cas du lymphoedème de la jambe, et ii) du différentiel de pressions entre pression de repos et pression de travail, les inventeurs ont réussi à mettre au point une nouvelle bande élastique aux propriétés de contention optimisées qui permet une meilleure conservation des pressions que les systèmes les plus performants, et dont le différentiel de pression ne diminue plus au cours du temps, mais au contraire augmente.

L'assemblage d'un matériau élastique avec un non tissé très spécifique fabriqué à partir de fibres conjuguées courtes, qui ont été frisées, permet d'obtenir une nouvelle bande élastique aux propriétés remarquables, en particulier pour le traitement des pathologies d'origine veineuse et les lymphoedèmes.

Des non tissés extensibles sont décrits dans la demande de brevet WO 2008/015972, pour leur utilisation comme bande de maintien ou de bandage,. Les bandes décrites dans ce document sont néanmoins trop fragiles pour que leur application comme bande de contention puisse être envisagée. Une bande de contention doit en effet être suffisamment solide pour résister à des déformations, des frottements et des tensions répétées pendant plusieurs jours, principalement au niveau du talon et de la malléole. Les bandes de maintien constituées des non tissés décrites dans la demande WO 2008/015972 sont trop facilement perforables et ne peuvent être utilisées dans le domaine de la thérapie compressive comme bandes de contention.

Ces non tissés ne correspondent ni à des bandes à allongement long ni à des bandes à allongement court. En effet, si on tente de mesurer leur allongement selon la norme EN 14704-1 qui caractérise les bandes de contention, ils cassent dès le premier cycle avant d'avoir atteint la force de traction maximale de 6 N/cm.

Le déposant a obtenu, par l'assemblage d'un tel non tissé et d'un matériau élastique, un produit qui présente des propriétés remarquables qui le rendent particulièrement adapté à une utilisation en thérapie compressive.

Le produit de l'invention permet d'obtenir avec une seule bande des résultats supérieurs - en termes de maintien de la pression appliquée et du différentiel de pression dans le temps - aux systèmes de contention aujourd'hui utilisés, et en particulier par rapport aux systèmes bicouches qui sont les plus performants.

Contrairement à ce que l'on aurait pu attendre, vu la fragilité de ces non tissés spécifiques, on constate que l'on peut assembler ces derniers avec un autre matériau sans les déstructurer ou les fragiliser encore plus, mais aussi sans détériorer leurs propriétés d'extensibilité et d'autoadhérence.

Cette nouvelle bande de contention pourra être utilisée seule ou en association avec une seconde bande dans un système de contention bicouche, selon la durée du traitement et la nature de la pathologie à traiter.

La demande US 2003/040691 décrit un bandage élastique comportant une bande non élastique non tissé absorbante, un voile non-tissé perméable à l'air non-élastique, et un matériau élastomère filé par fusion disposée entre les deux nappes.

Le matériau élastomère filé par fusion peut comprendre une pluralité de filaments en élastomère filé par fusion alignés dans la distribution sensiblement parallèle à une direction de la machine, les filaments fixés à un côté de la nappe non tissée non élastique et perméable à l'air sur un côté de la nappe non élastique non tissé absorbant.

La demande US 2011/208101 se rapporte à un pansement de compression destiné à fournir des propriétés de transport de l'humidité à une surface de contact.

Le pansement de compression est composé d'au moins une couche de tissu de transport de l'humidité constituée de fibres élastiques qui présentent un taux d'élasticité suffisant pour appliquer une force de compression sur une partie du corps. Le pansement compressif permet également de gérer des fluides et comprend une couche réservoir d'absorption qui est appliquée à la surface extérieure de la toile de transport de l'humidité. Cette couche réservoir absorbant peut être retirée en fonction des besoins sans perturber la force de compression appliquée à une partie du corps.

Un pansement poreux auto-adhésif élastique a été décrit dans le brevet US 5 393 339 comme moyen de compression enroulé autour d'une plaie et moyen d'absorption des fluides et exsudats de la plaie. Une forme de réalisation préférée du pansement est constituée d'un substrat élastique auto-adhésif qui ne colle pas aux vêtements fixé à une couche absorbante.

Dans le brevet FR 2 890 854 a été décrit un dispositif comprenant une première nappe de matériau souple, et une structure saillante solidaire de la nappe et comprenant une pluralité d'éléments saillants discrets. Ces éléments saillants comprennent des morceaux de mousse espacés les uns des autres et solidaires de ladite première nappe. Ce dispositif est utilisé pour le traitement de divers types d'oedèmes et d'infiltrations tissulaires excessives, ainsi que pour la réparation sportive.

La présente invention est donc relative à une nouvelle bande de contention comprenant un matériau élastique et un non tissé de fibres frisées obtenues à partir de fibres courtes conjuguées, ledit matériau élastique et ledit non tissé étant assemblés l'un à l'autre, et ledit non tissé ayant un grammage compris entre 70 g/m² et 300 g/m².

Les fibres sont avantageusement frisées de façon uniforme dans le sens de l'épaisseur du non tissé, et présentent un rayon de courbure moyen compris entre 10 et 200 micromètres.

Le nombre de fibres frisées à la surface du non-tissé est de préférence supérieur à 10 fibres frisées/cm².

Dans un mode de réalisation particulier, l'invention a pour objet une bande de contention comprenant un matériau élastique et un non tissé de fibres frisées obtenues à partir de fibres courtes conjuguées,
- ledit matériau élastique et ledit non tissé étant assemblés l'un à l'autre,
- ledit non tissé ayant un grammage compris entre 70 g/m² et 300 g/m²,
- lesdites fibres frisées étant frisées de façon uniforme dans le sens de l'épaisseur du non tissé, et présentant un rayon de courbure moyen compris entre 10 et 200 micromètres, et le nombre de fibres frisées à la surface du non-tissé étant supérieur à 10 fibres frisées/cm².

Selon une variante, la présente invention est relative à une bande de contention autoadhérente dans laquelle les boucles du non tissé après aiguilletage dépassent de la surface du matériau élastique rendant ce dernier autoadhérent. La bande peut être élastique et autoadhérente sans contenir du latex. Avantageusement, la bande peut contenir moins de 0.01% en poids de latex ou d'un adhésif de faible adhérence, tout en étant auto-adhérente.

Une telle bande autoadhérente est caractérisée en ce qu'elle comprend un matériau élastique et un non tissé de fibres frisées obtenues à partir de fibres courtes conjuguées, ledit matériau élastique et ledit non tissé étant aiguilletés l'un à l'autre, lesdites fibres étant frisées de façon uniforme dans le sens de l'épaisseur du non tissé, et présentent un rayon de courbure moyen compris entre 10 et 200 micromètres, le nombre de fibres frisées à la surface du non-tissé étant supérieur à 10 fibres frisées/cm², et la bande présente une force de pelage à 180° sur elle-même comprise entre 0,02 N/cm et 0,5 N/cm et de préférence entre 0,025 et 0,05 N/cm.

Selon une autre variante, la présente invention est relative à une bande de contention dans laquelle quelques boucles de non tissé apparaissent en surface du matériau élastique après aiguilletage, de telle sorte que le glissement de la bande sur elle-même soit diminué.

Une telle bande est caractérisée en ce qu'elle comprend un matériau élastique et un non tissé de fibres frisées obtenues à partir de fibres courtes conjuguées, ledit matériau élastique et ledit non tissé étant aiguilletés l'un à l'autre, lesdites fibres étant frisées de façon uniforme dans le sens de l'épaisseur du non tissé, et présentant un rayon de courbure moyen compris entre 10 et 200 micromètres, le nombre de fibres frisées à la surface du non-tissé étant supérieur à 10 fibres frisées/cm², et la surface du matériau élastique présente après assemblage un coefficient de frottement dynamique compris entre 2 et 4 et de préférence entre 3 et 4.

Le non tissé et le matériau élastique peuvent être assemblés sur l'ensemble de leur surface, de préférence sur l'ensemble de la surface du matériau élastique; la superficie et la forme de leurs surfaces peuvent être identiques ou différents. Dans le cas où la bande n'est pas autoadhérente et utilisée seule, on préférera que la surface du non tissé soit supérieure à celle du matériau élastique, en particulier on préférera une longueur supérieure du non tissé, de manière à permettre la fixation terminale de la bande sur elle-même à la fin de l'enroulement grâce à l'autoadhérence du non tissé sur lui-même.

Le non tissé peut avoir une largeur identique à celle du matériau élastique et une longueur supérieure, de telle sorte que les largeurs des deux matériaux se recouvrent, mais qu'une partie de la longueur du non tissé ne soit pas recouverte par le matériau élastique afin de faciliter la fin de la pose de la bande par l'adhérence du non tissé sur lui-même.

Des non tissés utilisables dans le cadre de la présente invention sont décrits dans la demande de brevet WO 2008/015972.

De façon générale, les fibres qui ont été utilisées pour fabriquer le non tissé sont de préférence conjuguées, de nature polymérique, et non continues (courtes).

Les fibres conjuguées au sens de l'invention sont des fibres «à frisabilité latente» dotées d'une structure asymétrique ou stratifiée, qui ont la propriété de friser sous l'effet d'un chauffage. Elles doivent cette propriété à la différence de coefficient de contraction thermique des polymères qui les constituent.

Ces fibres sont avantageusement constituées d'au moins deux polymères qui présentent un coefficient de contraction thermique différent. Ces polymères ont ordinairement des points de ramollissement ou des points de fusion différents. Ils peuvent être choisis parmi les polymères thermoplastiques comme par exemple : les polymères oléfiniques (notamment les polymères de polyoléfines C₂₋₄ tels que les polyéthylènes et polypropylènes basse, moyenne et haute densité), les polymères acryliques (notamment les polymères acrylonitriliques à unités acrylonitrile tels que les copolymères acrylonitrile/chlorure de vinyle), les polymères vinylacétaliques (notamment les polymères de polyvinylacétal), les polymères chlororovinyliques (notamment les polychlorures de vinyle, les copolymères chlorure de vinyle/acétate de vinyle et les copolymères chlorure de vinyle/acrylonitrile), les polymères chlorovinylidéniques (notamment les copolymères chlorure de vinylidène/chlorure de vinyle et les copolymères chlorure de vinylidène/acétate de vinyle), les polymères styréniques (notamment les polystyrènes résistants à la chaleur), les polymères polyesters (notamment les polymères de polyalkylène C₂₋₄ arylates tels que les polymères de polyéthylène téréphtalate, de polytriméthylène téréphtalate, de polybutylène téréphtalate et de polyéthylène naphtalate), les polymères polyamides (notamment les polymères polyamides aliphatiques tels que les polyamides 6, 6-6, 11, 12, 6-10 et 6-12, les polymères polyamides semi-aromatiques, les polymères polyamides aromatiques tels que le polyphénylène isophtalamide, le polyhexaméthylène téréphtalamide et le polyparaphénylène téréphtalamide), les polymères polycarbonates (notamment les polycarbonates de type bisphénol A), les polymères de polyparaphénylène benzobisoxazole, les polymères de polysulfure de phénylène, les polymères polyuréthanes, les polymères cellulosiques (notamment les esters de cellulose), etc. Ces polymères thermoplastiques peuvent éventuellement contenir d'autres unités copolymérisables.

Lorsque le chauffage des fibres est réalisé avec de la vapeur à haute température, selon le mode préféré de réalisation du non tissé, on préfère des polymères non adhésifs sous chaleur humide (ou des polymères hydrophobes ou non hydrosolubles résistants à la chaleur) de point de ramollissement ou de point de fusion supérieur ou égal à 100°C, comme par exemple les polymères polypropyléniques, les polymères polyesters et les polymères polyamides. Ces polymères permettent d'éviter le collage des fibres par fusion ou le ramollissement des fibres. On préfère tout particulièrement les polymères polyesters aromatiques et les polymères polyamides, pour leur excellente stabilité, leur résistance à la chaleur et leur aptitude à former des fibres.

Selon un mode préféré de la présente invention les fibres utilisées sont bicomposants. Les fibres bicomposants peuvent être composées de polymères de la même famille chimique ou de polymères de familles chimiques différentes, pourvu qu'ils aient des coefficients de contraction thermiques différents.

Dans un mode de mise en oeuvre, les fibres courtes conjuguées sont bicomposants, les deux composants les constituant étant des polymères qui présentent un point de ramollissement supérieur ou égal à 100°C, lesdits polymères étant choisis parmi les polymères polypropyléniques, les polymères polyesters et/ou les polymères polyamides, et de préférence sont deux polymères polyesters aromatiques différents.

On préfère que les fibres bicomposants soient constituées de deux polymères de la même famille chimique : par exemple d'un homopolymère et d'un copolymère. On peut en effet abaisser le taux de cristallinité de l'homopolymère, voire le rendre amorphe, ou abaisser son point de fusion ou son point de ramollissement, en copolymérisant le monomère avec un autre. L'écart de point de fusion ou de point de ramollissement des deux polymères peut être par exemple de l'ordre de 5 à 150°C, préférentiellement de 50 à 130°C, et plus préférentiellement de 70 à 120°C. La proportion de monomère copolymérisable, rapportée à la quantité totale de monomères, est par exemple de l'ordre de 1 à 50 % en moles, préférentiellement de 2 à 40 % en moles, et plus préférentiellement de 3 à 30 % en moles (particulièrement de 5 à 20 % en moles). Le rapport pondéral de l'homopolymère et du copolymère peut être choisi en fonction de la structure des fibres; il est par exemple, en termes de rapport homopolymère (A)/copolymère (B), de l'ordre de 90/10 à 10/90, préférentiellement de 70/30 à 30/70, et plus préférentiellement de 60/40 à 40/60. Dans un mode de réalisation préféré, les fibres bicomposants sont constituées de deux polymères polyesters aromatiques et en particulier de l'association d'un homopolymère de polyalkylène arylate (a) et d'un copolymère de polyalkylène arylate (b). L'homopolymère de polyalkylène arylate (a) peut être un homopolymère d'un acide dicarboxylique aromatique (notamment un acide dicarboxylique aromatique symétrique tel que l'acide téréphtalique ou l'acide naphtalène-2,6-dicarboxylique) et d'un composant alcane-diol (notamment l'éthylène-glycol ou le butylène-glycol). On utilise par exemple un polymère de la série des polyalkylène téréphtalates tel que le polyéthylène téréphtalate (PET) ou le polybutylène téréphtalate (PBT), et ordinairement un PET de viscosité intrinsèque de l'ordre de 0,6 à 0,7 servant à la fabrication des fibres de PET ordinaires. Le copolymère de polyalkylène arylate (b) peut être obtenu à partir d'un premier monomère servant à la préparation de l'homopolymère de polyalkylène arylate (a), et d'un deuxième monomère choisi parmi un acide dicarboxylique tel qu'un acide dicarboxylique aromatique asymétrique, un acide dicarboxylique alicyclique, un acide dicarboxylique aliphatique, un composant alcane-diol de chaîne plus longue que l'alcane-diol du polymère de polyalkylène arylate (a), et/ou un diol porteur d'une liaison éther.

Il est possible d'utiliser un seul ou d'associer plusieurs de ces deuxièmes monomères. Parmi ces composants, on utilise de préférence :
- un acide dicarboxylique aromatique asymétrique, notamment l'acide isophtalique, l'acide phtalique ou l'acide 5-sulfoisophtalique de sodium,
- ou un acide dicarboxylique aliphatique, notamment un acide dicarboxylique aliphatique C₁₋₁₂ tel que l'acide adipique,
- un alcane-diol, notamment le 1,3-propane-diol, le 1,4-butane-diol, le 1,6-hexane-diol ou le néopentylglycol,
- un polyoxyalkylène-glycol, notamment le diéthylène-glycol, le triéthylène-glycol, le polyéthylène-glycol ou le polytétraméthylène-glycol.

Parmi eux, on choisit de préférence notamment un acide dicarboxylique aromatique asymétrique tel que l'acide isophtalique, et un polyoxyalkylène-glycol tel que le diéthylène-glycol. Le copolymère de polyalkylène arylate (b) peut éventuellement être un élastomère à segments durs d'alkylène arylate (éthylène téréphtalate, butylène téréphtalate) et à segments souples par exemple de (poly)oxyalkylène-glycol. Dans le copolymère de polyalkylène arylate (b), la proportion de composant acide dicarboxylique destiné à abaisser le point de fusion ou le point de ramollissement rapportée à la quantité totale de composant acide dicarboxylique est par exemple de l'ordre de 1 à 50 % en moles, préférentiellement de 5 à 50 % en moles, et plus préférentiellement de 15 à 40 % en moles. La proportion de composant diol destiné à abaisser le point de fusion ou le point de ramollissement rapportée à la quantité totale de composant diol, est par exemple d'au plus 30 % en moles, et préférentiellement d'au plus 10 % en moles, par exemple de l'ordre de 0,1 à 10 % en moles.

La section transversale (perpendiculaire au sens de la longueur des fibres) des fibres bicomposants n'est pas limitée à la forme ronde (la forme ordinaire des fibres pleines) et aux formes modifiées (aplatie, elliptique, polygonale, 3- à 14-foliée, en T, en H, en V, en «os à chien» (en i), etc.), mais peut être aussi une section creuse. Toutefois, on choisit usuellement la section ronde.

Pour la structure transversale des fibres bicomposants, on peut citer les structures phasées formées par une pluralité de polymères, comme par exemple les structures de types coeur-écorce, îles et mer, mélangé, parallèle (côte-à-côte ou laminé multicouche), radial (laminé radial), radial creux, à blocs ou aléatoire. Parmi ces structures, on préfère, pour un développement plus spontané du frisage thermique, une structure de type coeur-écorce excentrique ou de type parallèle. Dans le cas de fibres bicomposants de type coeur-écorce et par exemple de type coeur-écorce excentrique, l'âme peut être constituée d'un polymère de la famille de l'alcool vinylique tel qu'un copolymère éthylène/alcool vinylique ou un alcool polyvinylique, ou d'un polymère thermoplastique de point de fusion ou de point de ramollissement bas, par exemple un polystyrène ou un polyéthylène basse densité, pourvu qu'elle autorise le frisage par le fait d'avoir un écart de coefficient de contraction thermique avec le polymère constituant l'écorce.

Dans un mode de réalisation particulier, les fibres bicomposants ont une structure de type côte à côte et sont constituées d'un premier polymère qui est un polyéthylène téréphtalate, et d'un second polymère qui est un copolymère d'un alkylène arylate avec l'acide isophtalique et/ou du diéthylène glycol.

Le titre moyen des fibres courtes conjuguées, notamment bicomposants, peut être par exemple compris entre 0,1 à 50 dtex, préférentiellement entre 0,5 et 10 dtex, et plus préférentiellement entre 1 et 5 dtex (particulièrement entre 1,5 et 3 dtex). Si le titre est trop fin, non seulement les fibres sont difficiles à fabriquer mais elles risquent de manquer de résistance. De plus, à l'étape de frisage, il est difficile d'obtenir de belles frisures en serpentin. Si le titre est trop gros, les fibres deviennent raides et rendent difficile le développement d'un frisage suffisant.

La longueur moyenne des fibres courtes conjuguées avant le frisage peut être par exemple comprise entre 10 et 100 mm, préférentiellement entre 20 et 80 mm, et plus préférentiellement entre 25 et 75 mm (particulièrement entre 40 et 60 mm). Si les fibres sont trop courtes, outre la difficulté de former le voile de fibres, l'enchevêtrement des fibres est insuffisant à l'étape de frisage et il est difficile de garantir de bonnes propriétés de résistance et d'extensibilité. Si les fibres sont trop longues, non seulement il devient difficile de former un voile de fibres de grammage uniforme, mais les fibres s'enchevêtrent excessivement lors de la formation du voile, au point de se gêner mutuellement au moment du frisage et d'empêcher le développement de l'extensibilité. De plus, dans l'invention, le choix de la longueur de fibre dans la plage précitée permet à une partie des fibres frisées à la surface du non-tissé d'émerger modérément de ladite surface du non-tissé et ainsi d'améliorer l'auto-adhésivité du non-tissé qui sera évoquée plus loin.

Dans un mode de réalisation, le titre moyen des fibres courtes conjuguées est compris entre 1 et 5 dtex, de préférence entre 1,5 et 3 dtex, et la longueur moyenne des fibres courtes conjuguées est comprise entre 10 à 100 mm, et de préférence entre 40 et 60 mm.

L'application d'un traitement thermique à ces fibres conjuguées a pour effet de développer le frisage et de leur imprimer des frisures en relief ayant la forme de serpentins (en spirale ou en «ressort à boudin»). Le rayon de courbure moyen des fibres frisées au sens de l'invention correspond au rayon de courbure moyen des cercles formés par les boucles des serpentins des fibres frisées ; il peut compris entre 10 et 200 microns, par exemple entre 10 et 250 microns, de préférence entre 20 et 200 microns, préférentiellement entre 50 et 160 microns, et plus préférentiellement entre 70 et 130 microns.

Le rayon de courbure moyen des fibres frisées peut être déterminé par microscopie électronique selon la méthode suivante. On prend une micrographie (grossissement x100) d'une section de non-tissé au microscope électronique à balayage (MEB). Parmi les fibres apparaissant sur le cliché, on sélectionne les fibres formant au moins 1 tour de spirale (serpentin) et on détermine leur rayon de courbure comme le rayon du cercle tracé le long de la spirale (rayon du cercle quand on observe la fibre frisée dans le sens de l'axe du serpentin). Quand la fibre dessine une spirale elliptique, le rayon de courbure est déterminé comme la demi-somme des grand et petit diamètres de l'ellipse. Afin d'exclure les fibres ayant développé un frisage en serpentin insuffisant et les fibres apparaissant elliptiques à cause d'une observation oblique de la spirale, on s'est limité aux fibres elliptiques de rapport entre grand et petit diamètres compris entre 0,8 et 1,2. On réalise la mesure sur l'image MEB d'une section arbitraire de non tissé et on détermine la moyenne sur une population de fibres n=100.

Lorsque le frisage est réalisé par de la vapeur à haute température, le non-tissé selon l'invention a pour caractéristique que le frisage des fibres conjuguées orientées à peu près parallèlement au sens planaire est développé de façon à peu près uniforme dans le sens de l'épaisseur. Dans une section de non tissé prise dans le sens de l'épaisseur, parmi les domaines délimités par un partage en trois parts égales dans le sens de l'épaisseur, le nombre de fibres formant au moins 1 tour de frisure spiralée est par exemple, dans la partie centrale (couche interne), de 5 à 50 par 5 mm (longueur en sens planaire) et 0,2 mm (épaisseur), préférentiellement de 10 à 50 par 5 mm (planaire) et 0,2 mm (épaisseur), et plus préférentiellement de 20 à 50 par 5 mm (planaire) et 0,2 mm (épaisseur).

Comme la majeure partie des fibres frisées ont leur axe orienté dans le sens planaire et que le nombre de frisures est uniforme dans le sens de l'épaisseur, le non-tissé manifeste une haute extensibilité (sans contenir de caoutchouc ou d'élastomère), et une bonne résistance opérationnelle (sans contenir d'adhésifs).

Dans la présente description, par «domaines délimités par un partage en trois parts égales dans le sens de l'épaisseur», on entend les différents domaines obtenus quand on coupe le non-tissé en trois tranches égales orientées perpendiculairement à l'épaisseur.

Dans le non-tissé, l'uniformité du frisage dans le sens de l'épaisseur peut être définie par le ratio d'incurvation de fibre. Par «ratio d'incurvation de fibre», on entend le rapport L2/L1 de la longueur de la fibre étirée bidimentionnellement L2 à la distance L1 des deux extrémités de la fibre à l'état frisé. Ce ratio d'incurvation de fibre (en particulier dans le domaine central dans le sens de l'épaisseur) est par exemple de l'ordre d'au moins 1,3 (par exemple de 1,35 à 5), préférentiellement de 1,4 à 4 (par exemple de 1,5 à 3,5), et plus préférentiellement de 1,6 à 3 (particulièrement de 1,8 à 2,5).

Lorsque le ratio d'incurvation de fibre est mesuré sur la base de micrographies électroniques de sections du non-tissé, la longueur de fibre L2 ne correspond pas à la longueur de la fibre qui serait obtenue si on étirait et rectilinéarisait la fibre frisée tridimensionnellement. Elle correspond à la longueur de fibre sur cliché qui est obtenue quand on étire et rectilinéarise la fibre apparaissant frisée bidimensionnellement sur le cliché. Autrement dit, la longueur de fibre sur cliché qui est mesurée selon l'invention est inférieure à la longueur de fibre réelle.

Lorsque le développement du frisage est à peu près uniforme dans le sens de l'épaisseur, le ratio d'incurvation de fibre est également uniforme dans le sens de l'épaisseur. L'uniformité du ratio d'incurvation de fibre peut être évaluée en comparant, dans une section prise dans le sens de l'épaisseur, les ratios d'incurvation de fibre obtenus dans les différentes couches délimitées par un partage en trois parts égales dans le sens de l'épaisseur. Ainsi, dans une section prise dans le sens de l'épaisseur, les ratios d'incurvation de fibre obtenus dans les différents domaines délimités par le partage en trois parts égales dans le sens de l'épaisseur se situent tous dans la plage précitée, et le rapport de la valeur minimale à la valeur maximale du ratio d'incurvation de fibre dans les différents domaines (rapport du domaine où le ratio d'incurvation de fibre est minimal au domaine où il est maximal) est par exemple de l'ordre d'au moins 75 % (par exemple de 75 à 100 %), préférentiellement de 80 à 99 %, et plus préférentiellement de 82 à 98 % (particulièrement de 85 à 97 %).

Selon un mode de réalisation, le non tissé présente dans une section prise dans le sens de l'épaisseur, un ratio d'incurvation de fibre supérieur ou égal à 1,3 dans chacun des domaines délimités par un partage en trois parts égales dans le sens de l'épaisseur, et le rapport de la valeur minimale à la valeur maximale du ratio d'incurvation de fibre dans les différents domaines est supérieur à 75 %.

Comme méthode concrète de mesure du ratio d'incurvation de fibre et de son uniformité, on peut appliquer la méthode qui consiste à prendre une micrographie de la section du non-tissé au microscope électronique et à mesurer le ratio d'incurvation de fibre sur des domaines choisis au sein des différents domaines du partage en trois parts égales dans le sens de l'épaisseur. La mesure est réalisée, dans chacune des couches supérieure (domaine recto), interne (domaine central) et inférieure (domaine verso), sur des domaines qui, dans le sens de la longueur, font au moins 2 mm, et dans le sens de l'épaisseur, sont positionnés près du centre de chaque couche et ont la même épaisseur d'un domaine à l'autre. De plus, ces domaines de mesure sont parallèles dans le sens de l'épaisseur, et sont définis de telle sorte que chacun renferme au moins 100 fragments de fibres autorisant la mesure de leur ratio d'incurvation (de l'ordre préférentiellement d'au moins 300, et plus préférentiellement de 500 à 1000). Après avoir défini ces domaines de mesure, on mesure le ratio d'incurvation de fibre de toutes les fibres situées dans le domaine et on calcule la valeur moyenne sur chaque domaine de mesure, puis on calcule l'uniformité du ratio d'incurvation de fibre en comparant le domaine montrant la valeur moyenne la plus grande et le domaine montrant la valeur moyenne la plus petite.

La mesure du ratio d'incurvation de fibre et de son uniformité peut être effectuée selon la méthodologie suivante. On prend une micrographie (grossissement x100) d'une section de non-tissé au microscope électronique et, dans une partie où apparaissaient les fibres sur le cliché, on partage l'épaisseur en trois domaines égaux (couches recto, interne et verso), et près du centre de chaque domaine, on définit des domaines de mesure d'au moins 2 mm dans le sens de la longueur et contenant au moins 500 fragments de fibres mesurables. Sur ces domaines, on mesure d'une part la distance inter-extrémités (distance la plus courte) entre les deux extrémités de la fibre et d'autre part la longueur de fibre (longueur de la fibre sur cliché).

Précisément, quand une extrémité de fibre émerge à la surface du non-tissé, elle est retenue telle quelle comme extrémité de mesure de la distance inter-extrémités; quand une extrémité de fibre plonge dans le non-tissé, on retient la partie limite de plongée dans le non-tissé (extrémité sur cliché) comme extrémité de mesure de la distance inter-extrémités.

Parmi les fibres imagées, on exclut de la mesure celles sur lesquelles on ne peut isoler une continuité d'au moins 100 µm. On calcule le ratio d'incurvation de fibre comme le rapport L2/L1 de la longueur de fibre L2 à la distance inter-extrémités L1 des fibres. Puis on calcule la moyenne sur chacune des couches recto, interne et verso du partage en trois parts égales dans le sens de l'épaisseur. On calcule enfin l'uniformité du ratio d'incurvation de fibre dans le sens de l'épaisseur à partir du rapport de ses valeurs maximale et minimale dans les différentes couches.

Le principe de la méthode de mesure de la longueur de fibre est illustré sur les figures 4-a et 4-b de la demande de brevet WO 2008/015972.

La Fig.4-(a) illustre le cas d'une fibre dont une extrémité émerge à la surface et l'autre extrémité plonge dans le non-tissé. La distance inter-extrémités L1 est ici la distance d'une extrémité de la fibre jusqu'à la partie limite de plongée dans le non-tissé. D'autre part, la longueur de fibre L2 est la longueur obtenue quand on étire bidimensionnellement, sur le cliché, la partie de la fibre observable (partie allant de l'extrémité de la fibre jusqu'à la partie de plongée dans le non-tissé).

La Fig.4-(b) illustre le cas d'une fibre dont les deux extrémités plongent dans le non-tissé. La distance inter-extrémités L1 est ici la distance des deux extrémités de la partie émergeant à la surface du non-tissé (extrémités sur cliché). D'autre part, la longueur de fibre L2 est la longueur obtenue quand on étire bidimensionnellement, sur le cliché, la fibre dans la partie émergeant à la surface du non-tissé.

Pour des fibres frisées en forme de serpentin, le pas moyen du serpentin est par exemple de l'ordre de 0,03 à 0,5 mm, préférentiellement de 0,03 à 0,3 mm, et plus préférentiellement de 0,05 à 0,2 mm.

Le non-tissé peut aussi contenir des fibres qui ne sont pas des fibres bicomposants. Parmi ces fibres additionnelles mono-composants, on peut par exemple citer les fibres de polymères déjà cités précédemment, mais aussi les fibres cellulosiques comme par exemple les fibres naturelles (laine de bois, laine de mouton, soie, chanvre), les fibres semi-synthétiques (notamment les fibres d'acétate telles que les fibres de triacétate) ou les fibres régénérées (rayonne, lyocell). Le titre moyen et la longueur moyenne des fibres mono-composants sont de préférence identiques à ceux des fibres bicomposants. On peut utiliser une seule espèce ou associer plusieurs espèces de ces fibres mono-composants. Parmi ces fibres mono-composants, la préférence va notamment aux fibres régénérées telles que les fibres de rayonne, aux fibres semi-synthétiques telles que les fibres d'acétate, aux fibres polyoléfiniques telles que les fibres de polypropylène ou de polyéthylène, aux fibres polyesters et aux fibres polyamides.

On préfère associer à des fibres bicomposants d'une famille chimique (par exemple de polyester) des fibres mono-composants de la même famille chimique.

Le rapport pondéral entre les fibres bicomposants et les fibres mono-composants est par exemple de l'ordre de 80/20 à 100/0 (par exemple de 80/20 à 99/1), préférentiellement de 90/10 à 100/0, et plus préférentiellement de 95/5 à 100/0.

Le non tissé qui compose la bande de l'invention est avantageusement dépourvu de fibres élastomériques. De telles fibres élastomériques sont généralement des filaments ou des fibres longues obtenus à partir de matériaux thermoplastiques tels que le polyuréthane, le polyamide, les copolymères du styrène, ou le polyester. Elles sont généralement obtenues par extrusion soufflage (« meltblown » en anglais) et ont généralement une longueur supérieure à 100 mm. Les non tissés sont avantageusement dépourvus de fibres longues disposées dans le sens longitudinal de la bande.

Le non-tissé peut aussi contenir des additifs tels que des agents stabilisants, des filtres UV, des photostabilisants, des antioxydants, des antibactériens, des désodorisants, des parfums, des colorants, des charges, des agents antistatiques, des retardateurs de flamme, des plastifiants, des lubrifiants, ou des retardateurs de cristallisation. On peut utiliser un seul ou plusieurs de ces additifs. Ces additifs peuvent aussi bien être supportés à la surface des fibres que contenus à l'intérieur des fibres.

Afin de pouvoir obtenir une bande de contention avec les propriétés recherchées, on choisira - parmi les non tissés réalisés à partir des fibres et des polymères précédemment décrits - un non tissé qui présente un grammage compris entre 70 et 300 g/m², de préférence compris entre 80 et 200 g/m², et de préférence encore entre 90 et 150 g/m².

Le grammage peut être mesuré selon la norme EN 9073-1.

Un grammage trop faible rend impossible l'assemblage car le produit trop fragile risque d'être détruit lors de l'assemblage, et un grammage trop élevé ne permet pas d'obtenir le compromis souhaité entre les caractéristiques d'extensibilité, de déchirabilité et d'autoadhérence.

Les autres propriétés mécaniques du non tissé seront de préférence les suivantes.

L'épaisseur du non tissé sera avantageusement comprise entre 0,25 et 5 mm, de préférence entre 0,4 et 2,5 mm et tout particulièrement entre 0,5 et 1,5 mm. L'épaisseur peut être mesurée selon la norme EN 9073-2.

L'élongation du non-tissé, c'est à dire son allongement à la rupture sera dans le sens longitudinal avantageusement compris entre 60 et 200% et de préférence entre 90 et 130 %, et dans le sens transversal compris entre 70 et 200 % et de préférence entre 60 et 160 %. L'élongation longitudinale et transversale peuvent être mesurées selon la norme EN 9073-3. Le test de cette norme consiste à mesurer l'allongement à la rupture, exprimé en pourcentage, qui correspond à la valeur de l'élongation. Les conditions de test dans le sens longitudinal sont les suivantes.

On soumet un échantillon du matériau à tester (par exemple le non tissé) de 300 mm de longueur et de 50 mm de largueur à un test de traction à l'aide d'un dynamomètre électronique dans lequel la traverse se déplace à une vitesse de 100 mm/mn. L'espace entre les mors est réglé à 200 mm et la largeur est celle de l'éprouvette soit 50 mm. Le dynamomètre s'arrête automatiquement quand l'échantillon subit sa rupture et l'appareillage enregistre l'allongement à la rupture. On reproduit le test sur trois échantillons et on prend la valeur moyenne.

Dans le sens transversal la mesure est réalisée de façon identique en adaptant la longueur entre les mors à la largeur du matériau à tester; par exemple avec un matériau de 10 cm de large, la longueur de l'échantillon entre les mors est de 6 cm.

L'élasticité du non tissé, telle que définie dans la norme EN 14704-1 c'est à dire sa récupération élastique, après un allongement de 30% est de préférence supérieure ou égale à 70% (par exemple comprise entre 70 et 100%) et de préférence comprise entre 80 et 95 %.

Le principe de la norme EN 14704-1 est basé sur la mesure de la récupération élastique est le suivant. Les conditions de la mesure sont les suivantes.

Une éprouvette (par exemple de bande de contention ou de non tissé) de 50 mm de largeur et de 200 mm de longueur est insérée dans les mors d'un dynamomètre électronique, lequel va effectuer une série de 5 cycles, jusqu'à un allongement de 30%, de traction «charge-décharge» à une vitesse de 100 mm/mn. L'allongement recouvré obtenu au cinquième cycle, exprimé en pourcentage, tel que défini dans la norme est automatiquement mesuré par le dynamomètre. La mesure de la récupération élastique, exprimée en pourcentage, est calculée selon la formule définie dans la norme sur la base de cet allongement recouvré. L'opération est répétée sur trois éprouvettes et on prend la valeur moyenne.

Dans le cadre de la présente invention on considère qu'un matériau est élastique si sa récupération élastique est supérieure ou égale à 70%.

L'autoadhérence de la bande selon l'invention est obtenue grâce à la présence de nombreuses fibres à l'état partiellement libre à la surface des non tissés, les fibres de surface s'enchevêtrant mutuellement au moment de la superposition de la bande sur elle-même. Pour obtenir cette propriété d'autoadhérence sans altérer les propriétés de déchirabilité et d'extensibilité, le nombre de fibres frisées, notamment en forme de serpentin ou de boucle, à la surface du non tissé, est avantageusement supérieur à 10 fibres frisées/cm², et de préférence compris entre 10 et 50 fibres frisées/cm². Pour la réalisation d'une bande de contention, on préférera un nombre de fibres frisées à la surface du non tissé compris entre 10 à 35 fibres frisées/cm².

Selon un mode de réalisation particulier, le non tissé est constitué de fibres bicomposants côte à côte à base de polymères polyesters aromatiques, le non tissé a un grammage compris entre 90 et 150 g/m², et le nombre de fibres frisées à la surface du non tissé est compris entre 10 et 35 fibres frisées/cm².

Le nombre de fibres frisées à la surface du non tissé peut être déterminé comme suit.

On prend une micrographie (grossissement x100) de la surface du non-tissé au microscope électronique, et on compte le nombre de fibres frisées (fibres faisant au moins un tour de spirale en boucle ou de serpentins formés à la surface du non-tissé), sur une aire unitaire de 1 cm² de surface de fibres imagées. La mesure peut être effectuée en cinq endroits arbitraires, et on calcule le nombre moyen de fibres bouclées arrondie à l'unité la plus proche.

La caractérisation de l'autoadhérence du non tissé ou de la bande est évaluée par la mesure de la force de pelage d'un échantillon replié sur lui-même. Cette force de pelage varie entre 0,02 et 0,5 N/cm et de préférence entre 0,025 et 0,1 N/cm.

Le test de caractérisation de l'autoadhérence consiste à mesurer la force de pelage à 180° d'un échantillon de matériau à l'aide d'un dynamomètre électronique. Cette force de pelage représente la valeur de l'autoadhérence du matériau. Les conditions de réalisation de la mesure sont avantageusement les suivantes.

Un échantillon de matériau de 60 cm de longueur et de 5 cm de largueur est replié sur lui-même en laissant libre les extrémités qui serviront à le fixer dans les mors du dynamomètre électronique. Les deux faces sont mises en contact sous la pression d'un poids équivalent à 1 kgf/cm. On effectue le pelage en réglant le dynamomètre à une vitesse de 300 mm/mn. Le dynamomètre donne directement la force de pelage, exprimée en N/cm. On reproduit le test sur 3 échantillons et on prend la valeur moyenne.

Par matériau élastique, on entend tout matériau qui présente, après un allongement de 30%, une récupération élastique, telle que définie dans la norme EN 14704-1, supérieure ou égale à 70%. Un matériau élastique au sens de l'invention n'est pas un non tissé de fibres frisées tel que décrit précédemment.

Ce matériau élastique pourra permettre d'adapter, selon la pathologie visée ou les utilisations envisagées, les propriétés d'absorption, d'amortissement, de conformabilité, de rigidité ou d'occlusivité de la bande de contention.

Le matériau élastique peut être choisi parmi les matériaux textiles, les matériaux alvéolaires, les films ou leurs combinaisons.

Parmi les matériaux textiles, on pourra utiliser des matériaux à base de fibres synthétiques ou naturelles. On peut citer les tissés, les non tissés, les tricots, les tricots 3D et leurs combinaisons.

On pourra utiliser tout type de non tissé comme matériau élastique à l'exclusion des non tissés à base de fibres conjuguées courtes frisées utilisés pour l'autre couche d'assemblage.

On préférera l'utilisation de matériaux qui permettent d'améliorer les propriétés d'amortissement ou d'absorption, et tout particulièrement les matériaux alvéolaires et les tricots 3D.

Parmi les matériaux alvéolaires, on peut citer les mousses hydrophobes ou hydrophiles par exemple à base de de polyuréthane ou à base d'oléfines. On préférera en particulier dans le cas du traitement des ulcères de jambe des mousses hydrophiles absorbantes comme par exemple la mousse commercialisée sous la référence MCF.03 par la société Advanced Medical Solution (AMS). Comme mousse non absorbante on peut citer par exemple les mousses à base d'oléfines commercialisées par la société ALVEO sous la dénomination Alvéolit®.

Selon un mode de réalisation, le matériau élastique est une mousse hydrophobe ou hydrophile, de préférence une mousse polyuréthane hydrophile, ou un tricot 3D.

Comme films on peut utiliser tout film polymérique souple par exemple à base de polyuréthane, polyoléfine, polyamide, polyester ou polychlorure de vinyle.

Parmi les tricots 3D peut citer, par exemple, les produits commercialisés par la société Louis Vuidon.

Le matériau élastique pourra contenir éventuellement des agents actifs qui contribuent à l'amélioration de la cicatrisation de l'ulcère de jambe ou qui permettent de diminuer la douleur ou l'oedème, ou encore des agents antibactériens.

Selon une variante de réalisation, dans le cas d'un matériau textile, on pourra introduire dans le matériau élastique des fibres antibactériennes, par exemple des fibres argent, ou imprégner celle-ci avec un antibactérien par exemple du triclosan.

La bande de contention selon l'invention est réalisée par l'assemblage du matériau élastique et d'un non tissé choisi parmi ceux tels que précédemment définis.

Par assemblage, on entend tout moyen permettant de lier le non tissé et le matériau élastique ensemble, si bien que leur simple superposition ne peut être considérée comme un assemblage.

Des technologies textiles variées, comme par exemple la couture, l'aiguilletage, la soudure par ultrasons, le complexage ou la fixation à l'aide d'un adhésif peuvent être utilisées pour réaliser cet assemblage.

Selon un mode de réalisation, le non tissé et le matériau élastique sont assemblés par aiguilletage, avec un adhésif ou par ultra-sons.

Ces technologies seront choisies en fonction de la nature des matériaux (non tissé et matériau élastique) à assembler en particulier leur résistance à la température et leur résistance mécanique.

Cet assemblage sera réalisé de manière à ne pas altérer les propriétés d'autoadhérence et d'extensibilité du non tissé, et à garantir l'absence de délaminage du produit au cours du temps.

Afin de garantir l'absence de délaminage du produit on s'assurera que la force de délaminage entre le non tissé et le matériau élastique soit supérieure à 10 cN/cm, et de préférence supérieure à 25 cN/cm.

Le principe de la mesure de délaminage est basé sur la méthode communément appelée décomplexage en T dans laquelle on mesure la force nécessaire pour délaminer les matériaux composant la bande de contention. La mesure de cette force de délaminage peut être réalisée selon le protocole suivant. On découpe une éprouvette de bande de contention de 50 mm de largeur et 300 mm de longueur. On délamine manuellement l'extrémité de cette éprouvette sur une longueur de 1 à 3 cm de manière à fixer chaque extrémité délaminée de la bande dans les mors d'un dynamomètre. La mesure est réalisée de telle sorte que l'on a un angle de 90° entre la bande de contention et l'extrémité de la bande préalablement délaminée. La force de délaminage est mesurée à l'aide du dynamomètre électronique dans lequel la traverse est mobile et se déplace à la vitesse de 300 mm/mn. Le dynamomètre enregistre directement cette force mesurée qui est exprimée en cN/cm. On reproduit le test sur 3 éprouvettes et on prend la valeur moyenne.

On préférera tout particulièrement un assemblage par aiguilletage qui permet d'obtenir un plus grand nombre de catégories de bandes de contention que les autres techniques.

En effet en jouant sur la nature ou les caractéristiques du matériau élastique, par exemple son épaisseur, et sur les paramètres d'aiguilletage (nombre d'aiguilles, profondeur d'aiguilletage...) on peut obtenir une bande dans laquelle les boucles du non tissé ressortent à la surface du matériau élastique du côté opposé au non tissé spécifique.

On obtient ainsi deux catégories de bandes : des bandes autoadhérentes et des bandes qui présentent une légère accroche, quand on les enroule sur elles-mêmes.

Selon un mode de réalisation, le matériau élastique et le non tissé sont aiguilletés, et la bande présente une force de pelage à 180° sur elle-même comprise entre 0,02 et 0,5 N/cm et de préférence entre 0,025 et 0,05 N/cm.

On obtient ainsi des bandes de contention très intéressantes car elles sont autoadhérentes, grâce à l'enchevêtrement des boucles du non tissé et de celles qui dépassent de la surface du matériau élastique, sans qu'il soit nécessaire d'utiliser du latex ou un adhésif.

Dans le cas des bandes qui présentent une faible accroche, seules quelques boucles traversent le matériau élastique et affleurent à la surface de ce dernier lors de l'aiguilletage. Ces bandes sont caractérisées par une surface du matériau élastique après assemblage qui présente un coefficient de frottement dynamique compris entre 2 et 4, et de préférence entre 2 et 3. Selon un mode de réalisation, le matériau élastique et le non tissé sont aiguilletés et la surface du matériau élastique présente après assemblage un coefficient de frottement dynamique compris entre 2 et 4, et de préférence entre 2 et 3.

Le coefficient de frottement dynamique caractérise l'aptitude d'une surface à glisser sur elle-même. La mesure du coefficient dynamique est basée sur le principe décrit dans la norme EN ISO 8295. Elle consiste à mesurer la force pour déplacer sur un grand plateau à l'aide d'un dynamomètre électronique un patin carré en acier de 200 g de 63,5 mm de longueur et 63,5 mm de largueur, lequel est recouvert de la bande de contention coté matériau élastique vers l'extérieur, qui glisse sur le grand plateau qui est lui aussi recouvert sur l'ensemble de sa surface de la bande de contention de la même façon. Les deux surfaces du matériau élastique sont donc en contact l'une sur l'autre et le patin est tiré à la vitesse de 150 mm/min par le dynamomètre. Les bandes sont collées sur le patin en acier et sur le plateau à l'aide d'un adhésif double face. Le patin est relié par un câble au capteur de force du dynamomètre. La mesure du coefficient de frottement dynamique est le rapport entre la force de traction, exprimée en Newtons, mesurée par le dynamomètre et le poids du patin, exprimé en Newtons, soit 1,96 N tel que défini dans la norme. On réalise la mesure sur trois échantillons découpés sur la même bande et on prend la valeur moyenne.

La bande de contention de l'invention peut être fabriquée par un procédé de fabrication, dans lequel le matériau élastique et le non tissé de fibres frisées sont assemblés l'un à l'autre par aiguilletage, de préférence sans précontrainte du non tissé de fibres frisées, c'est-à-dire sans étirer le non tissé préalablement à l'aiguilletage.

On pourra utiliser comme machine à aiguilletage, une machine FEHRER dotée d'une planche comprenant entre 800 et 3000 aiguilles par mètre linéaire.

On choisira de préférence une vitesse sortie sur la ligne d'aiguilletage comprise entre 0,5 et 25 mètre/minute, une longueur de pénétration des aiguilles comprise entre 8 et 25 mm, et une densité d'aiguilletage comprise entre 10 et 50 coups/cm². Selon un mode de mise en oeuvre, la longueur de pénétration des aiguilles est comprise entre 10 et 25 mm.

Souvent pour de courtes durées de traitement par exemple de 1 à 3 jours, ou dans une optique de prévention, on utilise des bandes non autoadhérentes que l'on fixe à la fin de l'enroulement sur elles-mêmes à l'aide d'un ruban adhésif ou d'agrafes. Un des problèmes de ces bandes est que, lors de l'utilisation, les spires de la bande glissent sur elles-mêmes ce qui conduit à les détendre et à leur glissement les unes par rapport aux autres, et la bande finit par glisser sur la jambe et tomber.

Avec une bande qui présente comme caractéristique un coefficient de frottement dynamique tel celui de la surface du matériau élastique après assemblage défini ci-dessus ce phénomène de glissement des spires sur elles-mêmes et spires sur spires est diminué ou supprimé, et on améliore l'efficacité de ce type de bandes.

Toutes les catégories de bandes pourront être utilisées seules ou dans des systèmes multicouches, en particulier des systèmes bicouches, selon le but recherché.

Selon un mode de réalisation préféré, afin de favoriser une pose précise par le personnel soignant, la bande de contention sera munie d'un moyen d'étalonnage. Ce moyen d'étalonnage peut être visuel, comme par exemple un ensemble de pictogrammes, régulièrement espacés, imprimés sur la bande ou réalisés au moyen d'un système d'étalonnage. Des informations sur les allongements à la pose recommandés peuvent être fournies avec le moyen d'étalonnage. L'étalonnage peut être réalisé par le personnel soignant sous la forme d'un pochoir. Ce type de pochoir ou les explications nécessaires pour le fabriquer peuvent être incorporés dans le conditionnement de la bande. Un kit comprenant plusieurs bandes de constitutions différentes, de largeurs différentes, de longueurs différentes et/ou dotées d'étalonnages différents pour appliquer des pressions spécifiques pourra être utilisé.

Lorsque le kit est indiqué pour le traitement des ulcères de jambes, il pourra en outre comprendre un ou plusieurs pansements destinés à être posés sur la plaie avant la pose de la bande.

Outre l'utilisation pour le traitement et la prévention des pathologies d'origine veineuse, en particulier les ulcères de jambe, ou le traitement des lymphoedèmes, en particulier les lymphoedèmes de la jambe pour lesquels il faut appliquer des pressions très élevées, les produits selon l'invention pourront être utilisés, de préférence quand ils sont autoadhérents, dans toute application où la conservation d'une pression appliquée est importante.

On peut ainsi citer le traitement et la prévention des pathologies traumatiques, des lésions articulaires, tendineuses, osseuses ou musculaires.

L'invention a également pour objet l'utilisation d'une bande de contention telle que décrite précédemment dans un système de contention bicouche, et en particulier comme première couche.

La bande de contention de l'invention permet d'obtenir des pressions de travail moyennes, voire même élevées, qui se maintiennent au cours du temps. Jusqu'à présent il était impossible de maintenir des pressions de travail élevées stables au cours du temps avec les bandes à allongement court.

Les performances des bandes de contention de l'invention peuvent être évaluées en termes de pressions de travail et de repos appliquées et de différentiel de pression, au cours du temps, en utilisant la méthode et l'appareil de test in vitro décrit dans la demande de brevet WO 2007/113430 page 17 ligne 26 à page 19 ligne 18.

Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.

L'écart de temps entre les mesures de pression de travail et de pression de repos est de 5 secondes et la fréquence des mesures de ces deux paramètres successifs est de 0,2Hz.

Pour tester les bandes de contention selon l'invention on peut déterminer l'allongement à la pose de la bande en fonction de la pression de travail recherchée, par exemple à l'aide de la courbe traction rupture telle que définie dans la norme EN 9073-3.

Pour poser la bande de façon appropriée, on peut étalonner les bandes à l'aide d'un pochoir comme décrit dans la demande de brevet WO 2007/113340 page 13 ligne 18 à page 14 ligne 6. Si nécessaire on peut affiner la valeur du pourcentage d'allongement à la pose par quelques tests successifs.

La valeur «Pression Max à T0» correspond à la première pression de travail enregistrée immédiatement après la pose et «Delta à T0» correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose. On calcule la différence de chacune de ces deux valeurs entre T0 et T24 heures «Pression Max (T0 - T24h)» et «Delta (T0 - T24h)».

On calcule également la perte de pression de travail à 24 heures «perte de Pression Max T24» par rapport à la pression de travail à la pose en faisant le rapport de la variation «Pression Max (T0 - T24h)» et de la «Pression Max à T0».

Les bandes de contention selon l'invention présentent avantageusement une pression de travail à 24 heures («Pression Max à T24») qui varie de 30 à 100 mm de mercure, par exemple de 40 à 80 mm Hg ou de 60 à 100 mm Hg.

En outre, les bandes de contention de l'invention présentent une excellente conservation de la pression appliquée à la pose au bout de 24 heures. La chute importante de cette valeur est généralement de l'ordre de 25 à 40 % au bout de 24 heures pour les bandes de contention à allongements courts de l'art antérieur, et de l'ordre de 20 à 25% pour les systèmes bicouches de l'art antérieur. Ainsi, la valeur «Pression Max (T0 - T24h)» des bandes de contention de la présente invention est avantageusement inférieure à 20%, par exemple comprise entre 10 et 15 %, voire même inférieure à 5 %.

Des bandes de contention de l'invention telles que la pression à 24 heures («Pression Max à T24») est supérieure à 60 mm de mercure, et de préférence comprise entre 70 et 100 mm de mercure, sont utiles pour le traitement des lymphoedèmes, en particulier de la jambe.

La valeur des différentiels de pression à 24 heures («Delta à T24») des bandes de l'invention est avantageusement entre 10 et 40 mm de mercure, par exemple entre 30 et 35 mm de mercure.

Les bandes de l'invention présente de façon tout à fait inattendue une différence entre le Delta après la pose et le Delta à 24 heures («Delta (T0 - T24h)») négative. Ce résultat est d'autant plus remarquable qu'il est obtenu par une baisse de la pression de travail « Pression Max » moins rapide que celle de la pression de repos. Les bandes de contention selon l'invention sont donc les premières à améliorer leur efficacité au cours du temps.

Les bandes de l'invention permettent de traiter les ulcères mixtes, artériels et veineux en appliquant des pressions de travail faibles de l'ordre de 30 à 35 mm de mercure et en maintenant un différentiel de pression allant de 5 à 20 mm de mercure.

Les bandes l'invention peuvent avoir un allongement supérieur à 100%, notamment lorsqu'il est mesuré selon la norme EN 14704-1. Ainsi, les bandes de l'invention peuvent présenter à la fois l'élasticité d'une bande à allongement long de l'art antérieur et un différentiel de pression à 24 heures très élevé caractéristique d'une bande à allongement court de l'art antérieur.

Les bandes l'invention peuvent avoir un allongement inférieur à 100%, notamment lorsqu'il est mesuré selon la norme EN 14704-1.

La bande de contention de l'invention est avantageusement telle qu'à une pression de travail «Pression Max à T0» équivalente à celle d'un autre système de contention de l'art antérieur, la perte de pression à T24 est diminuée, ce qui permet de changer la bande moins souvent.

On donnera maintenant divers exemples de bandes conformes à la présente invention.

### Exemples :

Différents matériaux ont été utilisés pour fabriquer des bandes.

### 1. Matériaux utilisés

### - a) matériaux élastiques

Les matériaux utilisés sont des produits commerciaux dont les dénominations ou références sont les suivantes et ils sont indiqués dans le tableau 1 en abrégé.
- mousses polyuréthane hydrophiles commercialisées par la société AMS d'épaisseur 4,5 mm (en abrégé Mousse 4,5 mm) et d'épaisseur 2,5 mm (en abrégé Mousse 2,5 mm).
- tricot 3D commercialisé par la société Louis Vuidon sous les références 9315 et 9031 (en abrégé Tricot 9315 et Tricot 9031).

### -b) Non tissé

Les exemples utilisent un non tissé autoadhérent, à base de fibres bicomposants asymétriques frisées, fabriqué selon l'enseignement de la demande de brevet WO 2008/015972 qui porte la référence SJJ 146 chez la société Kuraray .

La fibre utilisée est une fibre du type côte à côte à base de polyester dont la référence de la société Kuraray est PN- 780.

Ce non tissé présente les propriétés et les caractéristiques suivantes :
- Grammage (norme EN 9073-1) 134 g/m²
- Epaisseur (norme EN 9073-2) 1,14 mm
- Elasticité (norme EN 14704-1) 87 %
- Elongation longitudinale (norme EN 9073-3) 104 %
- Elongation transversale (norme EN 9073-3) 65 %
- Autoadhérence* 0,03 N/cm
- Nombre de fibres frisées
à la surface du non tissé** 27/cm²
* mesurée selon la méthode décrite précédemment
** mesuré selon la méthode décrite précédemment

### 2. Assemblage

Différentes techniques d'assemblage ont été utilisées pour fabriquer les bandes : l'aiguilletage, l'application d'un adhésif par points.

### a) Conditions d'assemblage par aiguilletage

Les essais de complexage par aiguilletage ont été réalisés sur une machine à aiguilletage FEHRER en utilisant une planche à 2500 aiguilles par mètre linéaire.

Deux types d'aiguilletage différents ont été mis en oeuvre pour réaliser les bandes de contention de l'invention.

### Aiguilletage 1

Les conditions de réalisation sur la ligne d'aiguilletage 1 sont les suivantes:
- Vitesse sortie sur la ligne d'aiguilletage : 1 mètre/minute
- Pénétration des aiguilles : 10 mm pour l'exemple 1 et 13 mm pour l'exemple 2
- Densité d'aiguilletage : 50 coups/cm²

L'association du matériau élastique et du non tissé avant aiguilletage 1 est mise en oeuvre sans précontrainte du non tissé.

### Aiguilletage 2

Les conditions de réalisation sur la ligne d'aiguilletage 2 étaient les suivantes:
- Vitesse ligne = 1 m/mm
- Pénétration des aiguilles : 13 mm
   Densité d'aiguilletage : 50 coups/cm²

L'association du matériau élastique et du non tissé avant aiguilletage 2 a été mise en oeuvre sans précontrainte du non tissé.

### b) Conditions d'assemblage par application d'adhésif à chaud par points avec un cylindre gravé

On a utilisé un assemblage avec un adhésif, lorsque la température d'application de l'adhésif nécessaire pour le déposer est compatible avec le non tissé. Lorsque la bande comprend une couche supplémentaire, la surface de la doit être suffisamment régulière pour que l'adhésif puisse être réparti de façon régulière.

Le produit a été réalisé sur une machine CAVIMELT de contrecollage par cylindre gravé (partie gauche).
- Cylindre utilisé = cylindre N°6 Gravure Netz 1
- Conditions d'essais de la machine :
   Vitesse de marche = 2 m/min
   Fente de laminage = 0,3 mm
   Pression cylindre de laminage = 3 bars
   Pression du contre cylindre = 2,5 bars
   Température de chauffe = 188°C
   Température de la colle = 180°C

On a encollé la couche supplémentaire de mousse, puis on est venu contrecoller le non tissé. L'adhésif hot melt utilisé était une colle polyester de dénomination commerciale GRILTEX D 2116 E® de la société EMS.

L'ensemble des bandes réalisées et les techniques d'assemblage sont rassemblées dans le tableau 1.

**Tableau 1**

| Exemple | Matériau élastique | assemblage |
|---|---|---|
| 1 | Mousse 4,5 mm | Aiguilletage 1 |
| 2 | Mousse 4,5 mm | Aiguilletage 1 |
| 3 | Mousse 4,5 mm | Adhésif |
| 4 | Mousse 2,5 mm | Aiguilletage 2 |
| 5 | TRICOT 9031 | Aiquilletage 2 |
| 6 | TRICOT 9315 | Aiguilletage 2 |

### 3. Performance des bandes de contention

Les performances des bandes de contention des Exemples 1 à 6 ont été évaluées en termes de pressions de travail et de repos appliquées et de différentiel de pression, au cours du temps.

On a utilisé la méthode et l'appareil de test in vitro décrit dans la demande de brevet WO 2007/113430 page 17 ligne 26 à page 19 ligne 18.

Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.

L'écart de temps entre les mesures de pression de travail et de pression de repos est de 5 secondes et la fréquence des mesures de ces deux paramètres successifs est de 0,2Hz.

Pour tester les bandes de contention selon l'invention on a déterminé l'allongement à la pose de la bande en fonction de la pression de travail recherchée, par exemple à l'aide de la courbe traction rupture telle que définie dans la norme EN 9073-3. D'après la loi de Laplace, l'allongement à effectuer correspond à la pression recherchée.

Pour poser la bande de façon appropriée, on a étalonné les bandes à l'aide d'un pochoir comme décrit dans la demande de brevet WO 2007/113340 page 13 ligne 18 à page 14 ligne 6. Si nécessaire on a affiné la valeur du pourcentage d'allongement à la pose par quelques tests successifs.

Chacune des bandes a été posée à un allongement donné, exprimé en pourcentage, qui est indiqué dans le tableau 2.

La valeur «Pression Max à T0» correspond à la première pression de travail enregistrée immédiatement après la pose et «Delta à T0» correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose. Puis on a calculé la différence de chacune de ces deux valeurs entre T0 et T24 heures «Pression Max (T0 - T24h)» et «Delta (T0 - T24h)».

On a aussi calculé la perte de pression de travail à 24 heures «perte de Pression Max T24» par rapport à la pression de travail à la pose en faisant le rapport de la variation «Pression Max (T0 - T24h)» et de la «Pression Max à T0».

On a comparé les performances des bandes selon l'invention aux systèmes de contention bicouches commercialisés par la société URGO Limited sous les dénominations K2® et K2 Lite®. Les bandes de ces produits commerciaux étaient déjà étalonnées.

L'ensemble des résultats a été reporté dans le tableau 2.

**Tableau 2**

| Exemple | Allongement à la pose | Pression max à T0 | Delta à T0 | Pression max à 24 h | Delta à 24 h | Delta (T0-T24) | Pression (T0-T24) | Perte de pression Max à T24 h |
|---|---|---|---|---|---|---|---|---|
| Ktech® | 55% | 31 | 15 | 23 | 12 | 3 | 8 | 25,8% |
| K2® | 55%+50% | 44 | 19 | 35 | 17 | 2 | 9 | 20,4% |
| K2 Lite® | 50%+50% | 33 | 10 | 25 | 8 | 2 | 8 | 24,2% |
| 1 | 30% | 77 | 28 | 67 | 30 | -2 | 10 | 12,9% |
| 2 | 30% | 87 | 32 | 76 | 34 | -2 | 11 | 12,6% |
| 3 | 30% | 69 | 15 | 58 | 16 | -1 | 11 | 15,9% |
| 4 | 30% | 34 | 12 | 34 | 16 | -4 | 0 | 0% |
| 5 | 30% | 42 | 8 | 37 | 13 | -5 | 5 | 11,9% |
| 6 | 30% | 45 | 13 | 44 | 16 | -3 | 1 | 2,2% |
| 4 +Kpress® | 30%+50% | 63 | 18 | 53 | 21 | -3 | 10 | 15,9% |

### Interprétation des résultats

L'analyse des résultats du tableau 2 démontre les performances des bandes de contention selon l'invention.

De façon générale ces résultats montrent que l'on peut traiter toutes les pathologies précédemment décrites car on a, selon les produits, une plage de valeur de la pression de travail à 24 heures qui varie de 34 à 76 mm de mercure.

On constate aussi que toutes ces bandes de contention présentent une excellente conservation de la pression appliquée à la pose au bout de 24 heures. La chute importante qui est généralement constaté pour les allongements courts, de l'ordre de 25 à 40 % au bout de 24 heures, et de l'ordre de 20 à 25% pour les systèmes bicouches qui sont les plus performants, est ici beaucoup plus faible car toujours inférieure à 20% et le plus souvent comprise entre 10 et 15 % voire même inférieure à 5 % pour le l'exemple 6 et nulle pour l'exemple 4.

Pouvoir appliquer une pression élevée et la conserver au cours du temps est un paramètre très important pour le traitement des lymphoedèmes, en particulier de la jambe, pour lesquels on souhaite une pression à 24 heures supérieure à 60 mm de mercure et de préférence comprise entre 70 et 100 mm de mercure.

Les exemples 1, 2 sont particulièrement bien adaptés pour ces pathologies.

De même on constate que la valeur des différentiels de pression à 24 heures varie entre 13 et 34 mm de mercure ce qui permet de s'adapter à toutes les catégories d'ulcères de jambes indiquées précédemment.

On constate aussi que le différentiel de pression entre pression de travail et pression de repos ne diminue pas au cours du temps mais au contraire, de façon inattendue, augmente.

Ainsi pour toutes les bandes la différence entre le Delta après la pose et le Delta à 24 heures est négative.

Ce résultat est d'autant plus remarquable qu'il est obtenu par une baisse de la pression de travail « Pression Max » (qui garantit l'efficacité du système) moins rapide que celle de la pression de repos.

Les bandes de contention selon l'invention sont donc les premières à améliorer leur efficacité au cours du temps.

On peut aussi constater que ces résultats sont obtenus avec des produits à base de matériaux différents ou assemblés selon des technologies différentes.

Ainsi si l'on vise une bande qui présente un différentiel de pression à 24 heures de l'ordre de 15 à 25 mm de mercure les bandes de contention des exemples 3, 4 et 6 peuvent remplir ce cahier des charges.

L'exemple 4 est particulièrement intéressant car cette bande peut être utilisée de plusieurs façons.

Ainsi pour pouvoir traiter les ulcères mixtes, artériels et veineux on souhaite, à cause de la composante artérielle, des pressions de travail faibles de l'ordre de 30 à 35 mm de mercure mais conserver un différentiel de pression élevé.

Avec les bandes actuelles, il est très difficile de combiner ces deux exigences. Pour obtenir ce résultat il a ainsi été nécessaire de développer un nouveau système de contention spécifique K2 Lite®.

On constate que la bande de l'exemple 4 posée à 30 % d'allongement permet d'obtenir des valeurs de pressions dans la fourchette recherchée et du même ordre que le K2 Lite® tout en conservant un différentiel de pression plus élevé que celui du K2 Lite®.

De même cette bande présente des valeurs proches de celles de la première bande du système K2®la bande Ktech®.

Si l'on associe la bande de l'exemple 6 posée à un allongement de 30% avec la seconde bande KPress® du système K2® posée à 50% comme dans le système K2®, on constate que l'on obtient un système bicouche avec une valeur de différentiel de pression à 24 heures supérieure à celle du K2®. Grâce à l'utilisation de cette première bande, on obtient un système bicouche dont l'efficacité s'améliore au cours du temps, puisque le Delta (T0-T24) est négatif.

Cet exemple 4 est aussi remarquable car, si on applique la norme EN 14704-1, on constate qu'il s'agit d'une bande à allongement long puisque son allongement est de 108%.

On a donc une bande à allongement long qui présente un différentiel de pression à 24 heures très élevé (16 mm de mercure) qui est de l'ordre de ceux d'un allongement court. De plus, l'exemple 4 présente une conservation de la pression de travail parfaite puisqu'elle ne diminue pas; sa variation est nulle 0%.

Tous les autres exemples ont des valeurs d'allongement inférieures à 100% mais ces dernières conservent voire augmentent le différentiel de pression ainsi que la pression de travail au cours du temps. Un tel comportement correspond à la fois à un allongement court et à un allongement long.

Ceci illustre parfaitement les avantages du non tissé à base de fibres courtes et frisées qui n'est pas utilisable seul comme bande de contention car il ne présente pas les propriétés d'une bande à allongement long, ni d'une bande à allongement court, mais qui permet d'obtenir en association avec un matériau élastique des bandes qui cumulent les avantages des deux types de bandes sans leurs inconvénients. Tout ceci en donnant des bandes dont l'efficacité s'améliore au cours du temps, autre caractéristique qui n'avait jamais été observée.

Enfin la bande de l'exemple 4 est intéressante car, après aiguilletage, des boucles du non tissé apparaissent en surface de la mousse ce qui permet d'obtenir une surface du matériau élastique, ici la mousse, qui présente un coefficient de frottement dynamique de 3,87 ce qui va diminuer sa glissance sur elle-même lors des mouvements.

Cette bande pourra donc, comme expliqué précédemment, être utilisée, selon le traitement visé, seule par exemple sur une courte durée de 1 à 3 jours.

On retrouve ce phénomène pour tous les autres exemples aiguilletés dans lesquels la pénétration des aiguilles est supérieure à 10 mm.

Pour l'exemple 1 avec une mousse de 4,5 mm d'épaisseur et une pénétration des aiguilles de 10 mm aucune boucle n'apparaît en surface de la mousse.

Ainsi pour les exemples 2, 5 et 6 on trouve respectivement des coefficients de frottement dynamique de 3,58, 3,62 et 3,79.

## Revendications

1. Bande de contention comprenant un matériau élastique et un non tissé de fibres frisées obtenues à partir de fibres courtes conjuguées,
- ledit matériau élastique et ledit non tissé étant assemblés l'un à l'autre,
- ledit non tissé ayant un grammage compris entre 70 g/m² et 300 g/m²,
- lesdites fibres frisées étant frisées de façon uniforme dans le sens de l'épaisseur du non tissé, et présentant un rayon de courbure moyen compris entre 10 et 200 micromètres, et le nombre de fibres frisées à la surface du non-tissé étant supérieur à 10 fibres frisées/cm².

2. Bande de contention selon la revendication 1, **caractérisée en ce que** les fibres courtes conjuguées sont bicomposants, les deux composants les constituant étant des polymères qui présentent un point de ramollissement supérieur ou égal à 100°C, lesdits polymères étant choisis parmi les polymères polypropyléniques, les polymères polyesters et/ou les polymères polyamides, et de préférence sont deux polymères polyesters aromatiques différents.

3. Bande de contention selon la revendication 2, **caractérisée en ce que** les fibres bicomposants ont une structure de type côte à côte et sont constituées d'un premier polymère qui est un polyéthylène téréphtalate, et d'un second polymère qui est un copolymère d'un alkylène arylate avec l'acide isophtalique et/ou du diéthylène glycol.

4. Bande de contention selon la revendication 1, **caractérisée en ce que** le titre moyen des fibres courtes conjuguées est compris entre 1 et 5 dtex, de préférence entre 1,5 et 3 dtex, et la longueur moyenne des fibres courtes conjuguées est comprise entre 10 à 100 mm, et de préférence entre 40 et 60 mm.

5. Bande de contention selon la revendication 1, **caractérisée en ce que** les fibres frisées présentent un rayon de courbure moyen compris entre 50 et 160 microns, et de préférence entre 70 et 130 microns.

6. Bande de contention selon la revendication 1, **caractérisée en ce que** le non tissé a un grammage compris entre 80 et 200 g/m² et de préférence entre 90 et 150 g/m².

7. Bande de contention selon la revendication 1, **caractérisée en ce que** le nombre de fibres frisées à la surface du non tissé est compris entre 10 et 50 fibres frisées/cm², et de préférence entre 10 et 35 fibres frisées/cm².

8. Bande de contention selon la revendication 1, **caractérisée en ce que** le non tissé présente dans une section prise dans le sens de l'épaisseur, un ratio d'incurvation de fibre supérieur ou égal à 1,3 dans chacun des domaines délimités par un partage en trois parts égales dans le sens de l'épaisseur, et le rapport de la valeur minimale à la valeur maximale du ratio d'incurvation de fibre dans les différents domaines est supérieur à 75 %.

9. Bande de contention selon la revendication 1, **caractérisée en ce que** le matériau élastique et le non tissé sont aiguilletés, et **en ce que** la bande présente une force de pelage à 180° sur elle-même comprise entre 0,02 et 0,5 N/cm et de préférence entre 0,025 et 0,05 N/cm.

10. Bande de contention selon la revendication 1, **caractérisée en ce que** le matériau élastique et le non tissé sont aiguilletés et la surface du matériau élastique présente après assemblage un coefficient de frottement dynamique compris entre 2 et 4, et de préférence entre 2 et 3.

11. Bande de contention selon la revendication 1, **caractérisée en ce que** le matériau élastique est choisi parmi les matériaux textiles, les matériaux alvéolaires, les films ou leurs combinaisons.

12. Bande de contention selon la revendication 10, **caractérisée en ce que** le matériau élastique est une mousse hydrophobe ou hydrophile, de préférence une mousse polyuréthane hydrophile, ou un tricot 3D.

13. Bande de contention selon la revendication 1, **caractérisée en ce que** le non tissé et le matériau élastique sont assemblés par aiguilletage, avec un adhésif ou par ultra-sons.

14. Bande de contention selon la revendication 1, **caractérisée en ce que** le non tissé est constitué de fibres bicomposants côte à côte à base de polymères polyesters aromatiques, le non tissé a un grammage compris entre 90 et 150 g/m², et le nombre de fibres frisées à la surface du non tissé est compris entre 10 et 35 fibres frisées/cm².

15. Système de contention bicouche comprenant une bande de contention selon l'une des revendications précédentes comme première couche.

## Patentansprüche

1. Stützbandage, umfassend ein elastisches Material und ein Vlies aus gekräuselten Fasern, die aus kurzen konjugierten Fasern erhalten werden,
- wobei das elastische Material und das Vlies miteinander verbunden sind,
- wobei das Vlies ein Flächengewicht zwischen 70 g/m² und 300 g/m² hat,
- wobei die gekräuselten Fasern einheitlich in die Richtung der Dicke des Vlieses gekräuselt sind und einen durchschnittlichen Krümmungsradius zwischen 10 und 200 Mikrometer aufweisen, und wobei die Anzahl von gekräuselten Fasern an der Oberfläche des Vlieses größer als 10 gekräuselte Fasern/cm² ist.

2. Stützbandage gemäß Anspruch 1, **dadurch gekennzeichnet, dass** die konjugierten kurzen Fasern Zweikomponentenfasern sind, wobei die zwei Komponenten, aus denen sie bestehen, Polymere sind, die einen Erweichungspunkt größer oder gleich 100°C aufweisen, wobei die Polymere unter den Polypropylen-Polymeren, den Polyester-Polymeren und/oder den Polyamid-Polymeren ausgewählt und vorzugsweise zwei unterschiedliche aromatische Polyester-Polymere sind.

3. Stützbandage gemäß Anspruch 2, **dadurch gekennzeichnet, dass** die Zweikomponentenfasern eine Seite-an-Seite-Struktur haben und von einem ersten Polymer, das ein Polyethylen-Terephthalat ist, und einem zweiten Polymer, das ein Compolymer eines Alkylenarylats mit der Isophthalsäure und/oder Diethylenglykol ist, gebildet sind.

4. Stützbandage gemäß Anspruch 1, **dadurch gekennzeichnet, dass** der durchschnittliche Titer der konjugierten kurzen Fasern zwischen 1 und 5 dtex, vorzugsweise zwischen 1,5 und 3 dtex, beträgt, und die durchschnittliche Länge der konjugierten kurzen Fasern zwischen 10 und 100 mm und vorzugsweise zwischen 40 und 60 mm beträgt.

5. Stützbandage gemäß Anspruch 1, **dadurch gekennzeichnet, dass** die gekräuselten Fasern einen durchschnittlichen Krümmungsradius zwischen 50 und 160 Mikrometer und vorzugsweise zwischen 70 und 130 Mikrometer aufweisen.

6. Stützbandage gemäß Anspruch 1, **dadurch gekennzeichnet, dass** das Vlies ein Flächengewicht zwischen 80 und 200 g/m² und vorzugsweise zwischen 90 und 150 g/m² aufweist.

7. Stützbandage gemäß Anspruch 1, **dadurch gekennzeichnet, dass** die Anzahl von gekräuselten Fasern an der Oberfläche des Vlieses zwischen 10 und 50 gekräuselte Fasern/cm² und vorzugsweise zwischen 10 und 35 gekräuselte Fasern/cm² beträgt.

8. Stützbandage gemäß Anspruch 1, **dadurch gekennzeichnet, dass** das Vlies in einem in Richtung der Dicke genommenen Querschnitt ein Faserkrümmungsverhältnis größer oder gleich 1,3 in jedem der Bereiche aufweist, die durch eine Teilung in drei gleiche Teile in Richtung der Dicke begrenzt sind, und dass das Verhältnis des Mindestwerts zum Höchstwert des Faserkrümmungsverhältnisses in den verschiedenen Bereichen größer als 75 % ist.

9. Stützbandage gemäß Anspruch 1, **dadurch gekennzeichnet, dass** das elastische Material und das Vlies genadelt sind, und dass die Bandage eine Abziehkraft auf 180° auf sich selbst zwischen 0,02 und 0,5 N/cm und vorzugsweise zwischen 0,025 und 0,05 N/cm aufweist.

10. Stützbandage gemäß Anspruch 1, **dadurch gekennzeichnet, dass** das elastische Material und das Vlies genadelt sind, und dass die Oberfläche des elastischen Materials nach der Verbindung einen dynamischen Reibungskoeffizienten zwischen 2 und 4 und vorzugsweise zwischen 2 und 3 aufweist.

11. Stützbandage gemäß Anspruch 1, **dadurch gekennzeichnet, dass** das elastische Material unter den Textilmaterialien, den Schaumstoffen, den Folien oder ihren Kombinationen ausgewählt ist.

12. Stützbandage gemäß Anspruch 10, **dadurch gekennzeichnet, dass** das elastische Material ein hydrophober oder hydrophiler Schaum, vorzugsweise ein hydrophiler Polyurethanschaum oder eine 3D-Wirkware, ist.

13. Stützbandage gemäß Anspruch 1, **dadurch gekennzeichnet, dass** das Vlies und das elastische Material durch Nadeln mit einem Haftmittel oder durch Ultraschall verbunden werden.

14. Stützbandage gemäß Anspruch 1, **dadurch gekennzeichnet, dass** das Vlies aus Seite-an-Seite-Zweikomponentenfasern auf Basis von aromatischen Polyester-Polymeren gebildet ist, das Vlies ein Flächengewicht zwischen 90 und 150 g/m² hat, und die Anzahl von gekräuselten Fasern an der Oberfläche des Vlieses zwischen 10 und 35 gekräuselte Fasern/cm² beträgt.

15. Zweischichten-Stützsystem, umfassend eine Stützbandage gemäß einem der vorhergehenden Ansprüche als erste Schicht.

## Claims

1. A compression bandage comprising an elastic material and a nonwoven of crimped fibers obtained from short conjugate fibers,
- said elastic material and said nonwoven being assembled together,
- said nonwoven having a grammage of between 70 g/m² and 300 g/m²,
- said crimped fibers being uniformly crimped in the thickness direction of the nonwoven, and exhibiting a mean curvature radius between 10 and 200 micrometers, and
- the number of crimped fibers at the surface of the nonwoven being greater than 10 crimped fibers/cm².

2. The compression bandage as claimed in claim 1, **characterized in that** the short conjugate fibers are bicomponent fibers that are made of two polymer components which exhibit a softening point greater than or equal to 100°C, and which are selected in the group consiting of polypropylene polymers, polyester polymers and polyamide polymers, and which are preferably two different aromatic polyester polymers.

3. The compression bandage as claimed in claim 2, **characterized in that** the bicomponent fibers have a of side by side type structure and are composed of a first polymer which is a polyethylene terephthalate and of a second polymer which is a copolymer of an alkylene arylate with isophthalic acid and/or diethylene glycol.

4. The compression bandage as claimed in claim 1, **characterized in that** the short conjugate fibers have a mean count between 1 and 5 dtex, preferably between 1.5 and 3 dtex, and a mean length between 10 and 100 mm and preferably between 40 and 60 mm.

5. The compression bandage as claimed in claim 1, **characterized in that** the crimped fibers exhibit a mean curvature radius between 50 and 160 microns and preferably between 70 and 130 microns.

6. The compression bandage as claimed in claim 1, **characterized in that** the nonwoven has a grammage of between 80 and 200 g/m² and preferably between 90 and 150 g/m².

7. The compression bandage as claimed in claim 1, **characterized in that** the number of crimped fibers at the surface of the nonwoven is between 10 and 50 crimped fibers/cm² and preferably between 10 and 35 crimped fibers/cm².

8. The compression bandage as claimed in claim 1, **characterized in that** each nonwoven exhibits, in a cross-section taken parallel to the thickness direction thereof,
- a fiber incurvation ratio greater than or equal to 1.3, and
- a ratio between the minimum value of the fiber incurvation ratio and the maximum value of the fiber incurvation ratio greater than 75%,
wherein said ratio and said fiber invurvation ratio are measured in three parts of the non woven, each part corresponding to one third thereof in a cross-section taken perpendicular to the thickness direction thereof.

9. The compression bandage as claimed in claim 1, **characterized in that** the elastic material and the nonwoven are needled and **in that** the bandage exhibits a peel strength at 180° on itself of between 0.02 and 0.5 N/cm and preferably between 0.025 and 0.05 N/cm.

10. The compression bandage as claimed in claim 1, **characterized in that** the elastic material and the nonwoven are needled and the surface of the elastic material exhibits, after assembling, a dynamic friction coefficient of between 2 and 4 and preferably between 2 and 3.

11. The compression bandage as claimed in claim 1, **characterized in that** the elastic material is chosen from textile materials, cellular materials, films or their combinations.

12. The compression bandage as claimed in claim 10, **characterized in that** the elastic material is a hydrophobic or hydrophilic foam, preferably a hydrophilic polyurethane foam, or a 3D knitwear.

13. The compression bandage as claimed in claim 1, **characterized in that** the nonwoven and the elastic material are assembled by needling, with an adhesive or by ultrasound.

14. The compression bandage as claimed in claim 1, **characterized in that** the nonwoven is composed of side by side bicomponent fibers based on aromatic polyester polymers, the nonwoven has a grammage of between 90 and 150 g/m² and the number of crimped fibers at the surface of the nonwoven is between 10 and 35 crimped fibers/cm².

15. Bilayer compression system comprising one compression bandage as claimed in one of the preceding claims as a first layer.
